# EUROPÄISCHE PATENTSCHRIFT

(11) **EP 1 669 045 B1**
(45) Veröffentlichungstag und Bekanntmachung des Hinweises auf die Patenterteilung: **11.04.2012**
(21) Anmeldenummer: 04029186.6
(22) Anmeldetag: 09.12.2004
(51) Int. Cl.: A61F 5/00

(54) **Magenband zur Behandlung von Fettleibigkeit**
Gastric band for the treatment of obesity
Bande gastrique pour le traîtement de l'obésité

(43) Veröffentlichungstag der Anmeldung: 14.06.2006
(73) Patentinhaber: AMI Agency for Medical Innovations GmbH, 6840 Götzis (AT)
(72) Erfinder: Catona, Antonio, Dott. Prof., 27100 Pavia (IT)
(74) Vertreter: Hofmann, Ralf U.

(56) Entgegenhaltungen:
- EP-A- 0 876 808
- WO-A-94/27504
- US-A1- 2004 143 342
- US-B2- 6 572 627

## Beschreibung

Die Erfindung betrifft ein Einrichtung zur Behandlung von Fettleibigkeit, welche ein ringförmig um den Magen legbares Bandteil mit einer Verschlusseinrichtung zum Verschließen des Magenbandes im ringförmig um den Magen gelegten Zustand und ein Hüllteil zum Umhüllen und Abstützen eines gegenüber dem angelegten Bandteil proximal liegenden Magenbereichs umfasst.

Ein Magenband mit einem ringförmig um den Magen legbaren Bandteil und einer Verschlusseinrichtung ist beispielsweise aus der EP 0 702 529 B1 bekannt. Das Magenband weist ein um den Magen im Bereich des Mageneingangs zu legendes Bandteil auf, welches eine längs verlaufende innere Hohlkammer besitzt. An den beiden Enden des Bandteiles sind erste und zweite Verschlussteile angebracht, die eine Verschlusseinrichtung des Magenbandes bilden. Zur Verengung des Durchlassquerschnittes der Magenbandöffnung des ringförmig geschlossenen Magenbandes wird die Hohlkammer des Bandteils mit einem Füllmedium befüllt, wobei die Menge des Füllmediums vom gewünschten Durchlassquerschnitt abhängt. Die Befüllung des Magenbandes mit dem Füllmedium erfolgt über einen mit dem Magenband über ein Röhrchen verbundenen Injektionsport, der unter die Haut des Patienten implantiert wird.

Bei implantierten Magenbändern kann es im Laufe der Zeit zu unterschiedlichen Komplikationen kommen. Eine dieser Komplikationen besteht darin, dass der vor dem Magenband, also proximal zum Magenband, liegende Bereich des Magens sich erweitert, mit für den Patienten nachteiligen Effekten, beispielsweise einer Verringerung der Effektivität des Magenbandes.

Eine Einrichtung der eingangs genannten Art ist aus der US 6,572,627 B2 bekannt. Das Magenband weist ein Bandteil und von diesem an beiden Enden abstehende Verschlussfortsätze auf. In der US 6,572,627 B2 ist weiters eine perforierte Folie beschrieben. Nachdem das Magenband um den Magen angelegt worden ist, wird diese Folie um einen proximal zum angelegten Magenband liegenden Abschnitt des Magens gewickelt und durch Vernähen der überlappenden Abschnitte fixiert. Weiters wird die Folie am Magenband festgenäht. Das so gebildete Hüllteil dient zur Begrenzung der Expansion dieses Magenabschnittes.

Nachteilig an dieser Einrichtung ist u.a. die komplizierte Implantation. Um einen korrekten Wert für das vom Hüllteil umschlossene Volumen festzulegen, wird vor dem Umwickeln des Magenabschnitts mit dem Hüllteil ein Ballon in den Magen eingeführt und auf eine geeignete Größe expandiert.

Aufgabe der Erfindung ist es, eine verbesserte Einrichtung der eingangs genannten Art bereitzustellen. Erfindungsgemäß gelingt dies durch ein Magenband mit den Merkmalen des Patentanspruchs 1.

Eine erfindungsgemäße Einrichtung,bei der das Bandteil und das an ihm befestigte Hüllteil als herstellerseitig vorgefertigte Einheit ausgebildet sind, bildet somit ein Magenband mit einem integrierten Hüllteil. Diese vorgefertigte Einheit ist als Ganzes in den Körper des Patienten implantierbar. Das Hüllteil ist vorzugsweise untrennbar mit dem Bandteil verbunden, d.h. nicht ohne Zerstörung der vorgefertigten Einheit entfernbar.

Durch die Erfindung wird eine einfache und zuverlässige Implantation erreicht, wobei die Gefahr von operativen Fehlern verringert wird. Ein erfindungsgemäßes Magenband ist in vorteilhafter Weise laparoskopisch anbringbar.

Das Hüllteil kann am Bandteil angeklebt sein. Auch eine einstückige Ausbildung des Hüllteils mit dem Bandteil (oder einem Teil hiervon) ist denkbar und möglich.

Das elastische Hüllteil stützt die von ihm umhüllte Magenwand elastisch ab. Beispielsweise können im Zuge der Implantation des Magenbandes, nachdem das Bandteil um den Magen gelegt worden ist und die Verschlusseinrichtung geschlossen worden ist, die einander nunmehr benachbart liegenden Enden des Hüllteils miteinander vernäht werden, sodass das Hüllteil eine Art umfangsgeschlossene Stützmanschette für den umhüllten Bereich der Magenwand und gegebenenfalls ebenfalls umhüllten ösophagealen-gastralen Übergang bildet. Auch können die Enden des Hüllteils nach dem Anlegen des Bandteils um den Magen und Verschließen der Verschlusseinrichtung einander überlappen und in überlappenden Abschnitten miteinander vernäht werden.

In einer weiteren Ausführungsvariante der Erfindung könnte das Hüllteil mit Verschlussteilen versehen sein, die nach dem Umhüllen des Magenbereichs verschließbar sind, wodurch vom Hüllteil eine umfangsgeschlossene Manschette gebildet wird.

Das Hüllteil weist günstigerweise über seine Ausdehnung eine Vielzahl von verteilten Durchtrittsöffnungen auf, deren Flächen in Summe einen Großteil der Gesamtfläche des Hüllteils ausmachen. In einer vorteilhaften Ausführungsform der Erfindung ist das Hüllteil netzartig ausgebildet, d. h. es besitzt gitterartig sich kreuzende Stränge, die an den Kreuzungspunkten miteinander verbunden sind. Zur elastischen Ausbildung des Hüllteils besteht dieses dabei aus einem elastischen Kunststoffmaterial.

Vorzugsweise weist das Hüllteil mindestens eine Fensteröffnung auf, welche dazu dient, nach dem Anlegen des Magenbandes Magengewebe, welches über das Bandteil gezogen wird (von der distal zum Bandteil liegenden Seite des Magens her), an unter der Fensteröffnung liegendem Magengewebe anzunähen. Einem Verrutschen des Magenbandes im Laufe der Zeit wird dadurch entgegengewirkt. Diese mindestens eine Fensteröffnung kann beispielsweise rechteckig, quadratisch oder kreisrund ausgebildet sein. Vorzugsweise ist sie mindestens so groß, dass ein Kreis mit einem Durchmesser von 0,5 cm in sie einschreibbar ist.

Weitere Vorteile und Einzelheiten der Erfindung werden im Folgenden anhand der beiliegenden Zeichnung erläutert. In dieser zeigen:
- Fig. 1: eine Seitenansicht eines Ausführungsbeispiels eines erfindungsgemäßen Magenbandes im geschlossenen Zustand;
- die Fig. 2 und 3: perspektivische Darstellungen des Magenbandes von Fig. 1 aus verschiedenen Blickwinkeln;
- Fig. 4: einen Schnitt von Fig. 1;
- Fig. 5 und Fig. 6: perspektivische Darstellungen des Magenbandes von Fig. 1 aus verschiedenen Blickwinkeln, im geöffneten Zustand der Verschlusseinrichtung;
- Fig. 7: eine schematische Darstellung des um den Magen gelegten Magenbandes mit angeschlossenen Injektionsport;
- Fig. 8: eine etwas modifizierte Ausführungsform eines Hüllteils im flach ausgelegten Zustand, in Ansicht;
- Fig. 9: eine schematische Darstellung der beiden Endbereiche (in gegenüberliegender Lage) einer weiteren Ausführungsform eines Hüllteils.

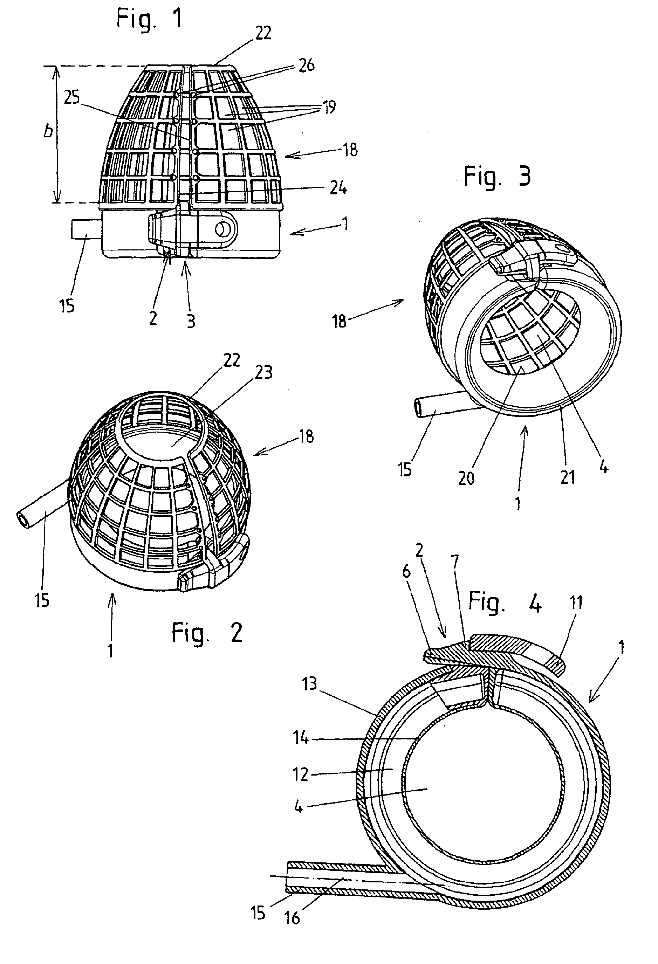

Das in den Fig. 1 bis 6 dargestellte Ausführungsbeispiel eines erfindungsgemäßen Magenbandes besitzt ein Bandteil 1, an dessen beiden Enden erste und zweite Verschlussteile 2, 3 angebracht sind, welche eine Verschlusseinrichtung zum Verbinden der beiden Endbereiche des Bandteils 1 darstellen. Im geschlossenen Zustand der Verschlusseinrichtung (Fig. 1 bis 4) wird ein umfangsgeschlossener Ring ausgebildet, der eine Magenbandöffnung 4 einschließt. Das erste Verschlussteil 2 besitzt einen über das stirnseitige Ende 5 des Bandteils 1 überstehenden Fortsatz 6, der mit einem Rastvorsprung 7 versehen ist. Das zweite Verschlussteil 3 wird von einem in der Nähe des anderen stirnseitigen Endes 8 gegenüber dem Bandteil 1 radial nach außen abstehenden Steg 9 gebildet, der eine Einstecköffnung 10 für den Fortsatz 6 aufweist. Zur Erleichterung des Schließens des Verschlusses ist am Steg 9 weiters eine Zuglasche 11 angebracht. Zum Schließen der Verschlusseinrichtung wird der Fortsatz 6 durch die Einstecköffnung 10 durchgezogen, bis der Rastvorsprung 7 durch die Einstecköffnung 10 durchgetreten ist und den Rand der Einstecköffnung 10 hintergreift.

Vorzugsweise sind der Fortsatz 6 mit seinem Rastvorsprung 7 und der Steg 9 aus einem Elastomer, insbesondere Silikon, mit einer solchen Härte und weiters mit einer solchen zusammenwirkenden Geometrie ausgebildet, dass die Verschlusseinrichtung bei einem Aufreißzug, der einen vorgegebenen Grenzwert überschreitet, sich öffnet. Es kann dadurch ein Sicherheitsverschluss bereitgestellt werden, durch welchen ein unzulässig hoher Druck im Magen vermieden werden kann.

Die an den beiden Enden des Bandteils angeordneten Verschlussteile 2, 3 können an Pfropfen angebracht sein, welche in die Enden des Bandteils 1 ragen und die Hohlkammer 12 des Bandteils 1 verschließen. Beispielsweise können die Pfropfen eingeklebt sein. Auch eine materialeinstückige Ausbildung der Verschlusseinrichtung oder Teilen hiervon mit dem Bandteil 1 ist denkbar und möglich. Das Bandteil 1 kann aus einem Elastomer, vorzugsweise aus Silikon bestehen.

Das Bandteil 1 wird im gezeigten Ausführungsbeispiel von einem im Bereich seiner beiden Enden verschlossenen Schlauch gebildet, in welchem die Hohlkammer 12 verläuft, welche somit im gezeigten Ausführungsbeispiel sich im Wesentlichen über die gesamte Länge des Bandteils 1 erstreckt. Der Rückenabschnitt 13 (das ist die die Hohlkammer 12 nach außen begrenzende Wand) des Bandteils 1 besitzt eine geringere elastische Dehnbarkeit als der innere Abschnitt 14. Dies kann beispielsweise durch eine dickere und/oder härtere Ausbildung des Rückenabschnitts erreicht werden. Zusätzlich oder stattdessen kann der Rückenabschnitt 13 auch durch eine Verstärkungslage verstärkt sein, die beispielsweise in das Material des Rückenabschnitts eingebettet ist oder auf dessen von der Hohlkammer 12 abgewandten Seite aufgeklebt ist und sich über die Länge des Bandteils 1 erstreckt. Diese Verstärkungslage kann hierbei in Längsrichtung und vorteilhafterweise auch in Querrichtung über ihre gesamte Ausdehnung durchgehende Fäden aufweisen und kann insbesondere als Rechteckgewebe, beispielsweise aus einem Kunststoffmaterial ausgebildet sein. Durch die weniger elastische oder unelastische Ausbildung des Rückenabschnitts wird dessen Ausdehnung bei der Befüllung des Bandteils 1 mit einem Füllmedium verringert bzw. verhindert und die Zugstabilität des Magenbandes gewährleistet.

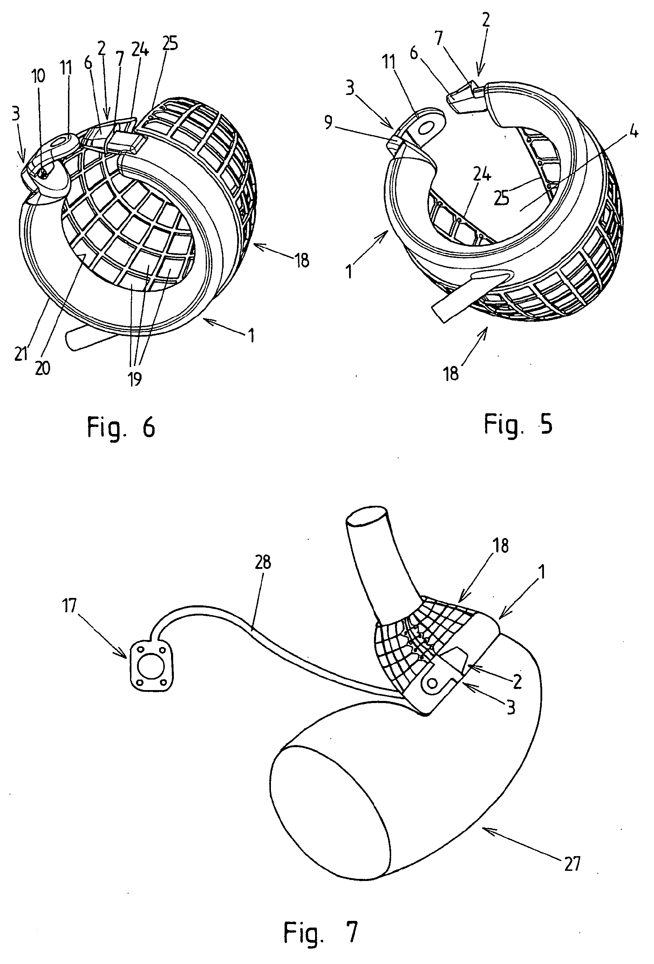

Das Magenband weist weiters ein Anschlussröhrchen 15 auf, dessen innerer Kanal 16 in die Hohlkammer 12 mündet. Das Anschlussröhrchen 15 dient zum Anschluss eines Injektionsports 17 (vgl. Fig. 7) an das Magenband.

Das Magenband weist weiters ein vom Bandteil 1 seitlich abstehendes elastisches Hüllteil 18 auf. Beispielsweise liegt die in Richtung senkrecht zur Längsausdehnung des Bandteils 1 gemessene überstehende Breite b des Hüllteils im Bereich zwischen 6 und 10cm. Das Hüllteil 18 ist ein flächiges Gebilde, d. h. seine Dicke, die weniger als 5mm, vorzugsweise weniger als 2mm beträgt, ist wesentlich geringer als seine Breite bzw. Länge. Im gezeigten Ausführungsbeispiel erstreckt sich das Hüllteil 18 über die Länge des Bandteils 1. Grundsätzlich wäre es auch denkbar und möglich, dass es über die stirnseitigen Enden des Bandteils 1 vorsteht, sodass sich im geschlossenen Zustand des Magenbandes überlappende Abschnitte des Hüllteils ausbilden.

Das Hüllteil 18 besitzt eine Vielzahl von über seine Ausdehnung verteilten Durchtrittsöffnungen 19. Das Hüllteil ist bevorzugterweise wie gezeigt netzartig (gitterartig) ausgebildet. Zur elastischen Abstützung der Magenwand besteht das Hüllteil aus einem Elastomer, beispielsweise aus Silikon, dessen Härte insbesondere im Bereich zwischen 30 und 60 Shore A liegen kann. Um einer Migration des Hüllteils in das Magengewebe entgegenzuwirken, kann das Hüllteil zumindest auf der im angelegten Zustand des Magenbandes der Magenwand zugewandten Seite, gegebenenfalls allseitig, mit Titan beschichtet sein. Die Beschichtungsdicke kann hierbei sehr klein sein und beispielsweise nur einige Atomlagen betragen.

Obwohl bevorzugt ist, dass das Hüllteil 18 nur über einen der beiden Längsränder 20, 21 des Bandteils 1 seitlich vorsteht, wäre es auch denkbar und möglich, ein beidseitig vorstehendes Hüllteil (welches einteilig ausgebildet ist oder aus zwei separaten, jeweils am Bandteils 1 befestigten Teilen besteht) vorzusehen, beispielsweise um einer Migration des Magenbandes in das Magengewebe stärker entgegenzuwirken.

Im gezeigten Ausführungsbeispiel ist das Hüllteil 18 im Bereich des Längsrandes 20 des Bandteils 1 an diesem angebracht, beispielsweise angeklebt.

Die Fig. 1 bis 6 zeigen das Magenband im nicht um den Magen angelegten Zustand. Hierbei weist das Hüllteil 18 im geschlossenen Zustand der Verschlusseinrichtung 2, 3 eine kuppel- bzw. domförmige Gestalt auf, d.h. die Seitenränder des Hüllteils weisen in Seitenansicht des Magenbandes gesehen (Fig. 1) einen bogenförmigen Verlauf auf, der sich bis zum vom Bandteil 1 abgelegenen freien Längsrand 22 des Bildteils erstreckt, wobei sich der Abstand von der Längsmittelachse des Magenbandes verringert (es wirkt hierbei keine äußere Kraft ein). Der vom Bandteil 1 abgelegene freie Längsrand 22 des Hüllteils 18 umschließt eine Öffnung 23, die vorzugsweise einen Durchmesser im Bereich zwischen 2 und 3 cm aufweist, wobei ein Wert von ca. 2,5 cm besonders bevorzugt ist.

Beim gezeigten Ausführungsbeispiel liegen die stirnseitigen Enden 24, 25 des Hüllteils 18 einander benachbart, wenn die Verschlusseinrichtung 2, 3 geschlossen ist, und können im um den Magen gelegten Zustand des Magenbandes miteinander vernäht werden, wobei im gezeigten Ausführungsbeispiel Nähösen 26 vorhanden sind.

Der um den Magen 27 angelegte Zustand des Magenbandes ist in Fig. 7 schematisch dargestellt. Das Bandteil 1 ist um den Magen 27 in einem proximalen Bereich desselben gelegt worden und die Verschlusseinrichtung 2, 3 ist geschlossen worden. Die beiden stirnseitigen Enden 24, 25 des Hüllteils 18 sind miteinander vernäht worden, sodass das Hüllteil 18 zumindest den gegenüber dem Bandteil 1 proximal (= zum Mund gerichtet) liegenden Bereich des Magens 27 manschettenartig umgibt. Auch der ösophageale-gastrale Übergang (in welchem innen der Cardia-Schließmuskel liegt) kann vom Hüllteil 12 umgeben sein. Die Elastizität in Kombination mit der geometrischen Ausgestaltung des Hüllteils 18 ist hierbei so gewählt, dass es nicht zu straff am Magen anliegt (andernfalls bestünde Migrationsgefahr), andererseits aber die Magenwand in ausreichendem Maß elastisch abstützt.

An das Magenband wird in der Folge mittels eines Verbindungsröhrchens 28 ein Injektionsport 17 angeschlossen, der unter der Haut des Patienten implantiert wird. Die Größe der Magenbandöffnung 4 kann dann durch Injektion von Füllmedium mittels einer Nadel in den Injektionsport 17 eingestellt werden.

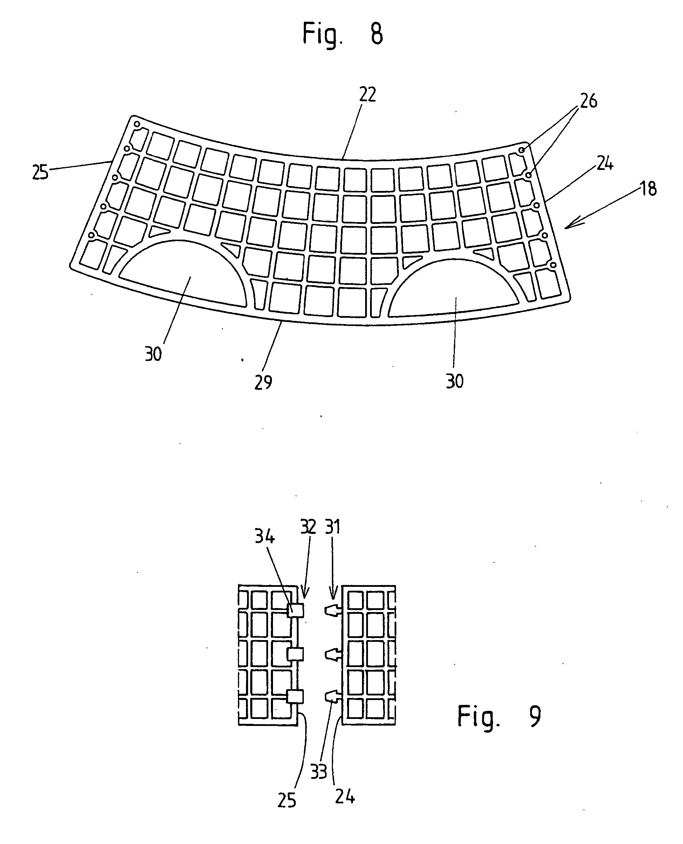

Ein weiteres Ausführungsbeispiel eines Hüllteils 18 ist in Fig. 8 dargestellt. Das Hüllteil 18 wird im Bereich seines Längsrands 29 an das Bandteil 1 angeklebt. In der Nähe des Längsrands 29 sind zwei Fensteröffnungen 30 ausgebildet. Nach dem Anlegen des Bandteils 1 um den Magen und Vernähen der beiden stirnseitigen Enden 24, 25 des Hüllteils 18 kann Magengewebe über das Bandteil 1 gezogen werden und durch die Fensteröffnung 30 mit unterhalb des Hüllteils 18 liegendem Magengewebe vernäht werden, um einem im Laufe der Zeit auftretenden Verrutschen des Magenbandes entgegenzuwirken.

Anstelle eines Vernähens der stirnseitigen Enden 24, 25 oder von überlappenden Abschnitten des Hüllteils 18 wäre es auch denkbar und möglich, das Hüllteil 18 im Bereich seiner stirnseitigen Enden oder seiner überlappenden Abschnitte mit einer Verschlusseinrichtung 31, 32 zu versehen, wie dies in Fig. 9 beispielhaft schematisch dargestellt ist. Beim in Fig. 9 schematisch dargestellten Ausführungsbeispiel sind am einen stirnseitigen Ende 24 des Hüllteils Fortsätze 33 mit Rastvorsprüngen angebracht, welche in Einstecköffnungen eingesteckt werden können, die in am anderen stirnseitigen Ende 25 angebrachten Stegen 34 angeordnet sind, wobei die Rastvorsprünge der Fortsätze 33 im eingesteckten Zustand die Ränder der Einstecköffnungen in den Stegen 34 hintergreifen. Auch andere Verschlusseinrichtungen wären denkbar und möglich, beispielsweise mittels hakenartigen Elementen.

Unterschiedliche Modifikationen der gezeigten Ausführungsbeispiele sind denkbar und möglich, ohne den Bereich der Erfindung zu verlassen. Beispielsweise könnte die Verschlusseinrichtung für das Bandteil auch in anderer Weise als gezeigt ausgebildet sein. Denkbar und möglich wäre es beispielsweise auch, das Hüllteil anstelle in Form eines Netzes bzw. Gitters als gelochte Folie auszubilden. Prinzipiell wäre es auch denkbar und möglich, das Bandteil ohne innere Hohlkammer auszubilden. Es könnte dabei auch vorgesehen sein, den Durchmesser der Magenbandöffnung mittels einer Kolben-Zylinder-Einheit zu verstellen, wobei die Kolbenstange des Kolbens im Bereich des einen Endes des Bandteils 1 und der Zylinder im Bereich des anderen Endes des Bandteils 1 befestigt ist und mindestens eine dieser Teile (Kolbenstange und Zylinder) erst nach dem Legen des Bandteils 1 um den Magen befestigt wird (beispielsweise mittels eines Schnappverschlusses). Die Stellung des Kolbens gegenüber dem Zylinder könnte wiederum mittels eines angeschlossenen Injektionsports eingestellt werden.

Denkbar und möglich wäre es auch, das erste Verschlussteil 2 und/oder das zweite Verschlussteil 3 mit Titan zu beschichten, um Verwachsungen in diesem Bereich hintan zu halten. Eine spätere Entfernung des Magenbandes wird dadurch erleichtert. Auch eine Beschichtung des Bandteils 1 mit Titan, insbesondere auf seiner dem Magen zugewandten Seite, ist denkbar und möglich, um einer Migration des Bandteils in das Magengewebe entgegenzuwirken. Titanbeschichtungen können überall dort vorgesehen werden, wo Verwachsungen unerwünscht sind. Die Beschichtungsdicke kann hierbei wiederum sehr klein sein und beispielsweise nur einige Atomlagen betragen.

### Legende zu den Hinweisziffern:

- 1: Bandteil
- 2: erstes Verschlussteil
- 3: zweites Verschlussteil
- 4: Magenbandöffnung

- 6: Fortsatz
- 7: Rastvorsprung

- 9: Steg
- 10: Einstecköffnung
- 11: Zuglasche
- 12: Hohlkammer
- 13: Rückenabschnitt
- 14: innerer Abschnitt
- 15: Anschlussröhrchen
- 16: innerer Kanal
- 17: Injektionsport
- 18: Hüllteil
- 19: Durchtrittsöffnung
- 20: Längsrand
- 21: Längsrand
- 22: freier Längsrand
- 23: Öffnung
- 24: stirnseitiges Ende
- 25: stirnseitiges Ende
- 26: Nähöse
- 27: Magen
- 28: Verbindungsröhrchen
- 29: Längsrand
- 30: Fensteröffnung
- 31: Verschlussteil
- 32: Verschlussteil
- 33: Fortsatz
- 34: Steg

## Patentansprüche

1. Einrichtung zur Behandlung von Fettleibigkeit, welche ein ringförmig um den Magen legbares Bandteil (1) mit einer Verschlusseinrichtung (2, 3) zum Verschließen des Magenbandes im ringförmig um den Magen gelegten Zustand und ein Hüllteil (18) zum Umhüllen und Abstützen eines gegenüber dem angelegten Bandteil (1) proximal liegenden Magenbereichs umfasst, **dadurch gekennzeichnet, dass** das Bandteil (1) und das am Bandteil (1) festgelegte und von diesem seitlich abstehende Hüllteil (18) als herstellerseitig vorgefertigte Einheit ausgebildet sind.

2. Einrichtung nach Anspruch 1, **dadurch gekennzeichnet, dass** das Hüllteil (18) eine Vielzahl von über seine Ausdehnung verteilten Durchtrittsöffnungen (19) aufweist, wobei die Summe der Flächen der Durchtrittsöffnungen (19) vorzugsweise einen Großteil der Gesamtfläche des Hüllteils (18) ausmacht.

3. Einrichtung nach Anspruch 2, **dadurch gekennzeichnet, dass** das Hüllteil (18) netzartig ausgebildet ist.

4. Einrichtung nach einem der Ansprüche 1 bis 3, **dadurch gekennzeichnet, dass** das Hüllteil (18) am Bandteil (1) angeklebt ist, vorzugsweise im Bereich eines der Längsränder (20, 21) des Bandteils (1).

5. Einrichtung nach einem der Ansprüche 1 bis 4, **dadurch gekennzeichnet, dass** die beiden stirnseitigen Enden (24, 25) des Hüllteils (18) im um den Magen gelegten Zustand des Magenbandes einander benachbart sind oder an die beiden stirnseitigen Enden (24, 25) anschließende Abschnitte des Hüllteils (18) einander überlappen und zur Ausbildung einer umfangsgeschlossenen Stützmanschette die einander benachbarten stirnseitigen Enden (24, 25) oder die an die beiden stirnseitigen Enden (24, 25) anschließenden überlappenden Abschnitte aneinander befestigbar sind.

6. Einrichtung nach Anspruch 5, **dadurch gekennzeichnet, dass** zur gegenseitigen Befestigung der stirnseitigen Enden (24, 25) oder der an die stirnseitigen Enden (24, 25) anschließenden überlappenden Abschnitte des Hüllteils (18) diese miteinander vemähbar sind, vorzugsweise durch Nähösen (26).

7. Einrichtung nach Anspruch 5 oder Anspruch 6, **dadurch gekennzeichnet, dass** zur gegenseitigen Befestigung der stirnseitigen Enden (24, 25) oder der an die stirnseitigen Enden (24, 25) anschließenden überlappenden Abschnitte des Hüllteils (18) eine Verschlusseinrichtung vorhanden ist, welche vorzugsweise von miteinander verrastbaren Verschlussteilen (31, 32) gebildet wird.

8. Einrichtung nach einem der Ansprüche 1 bis 7, **dadurch gekennzeichnet, dass** sich das Hüllteil (18) zumindest über die Länge des Bandteils (1) erstreckt.

9. Einrichtung nach einem der Ansprüche 1 bis 8, **dadurch gekennzeichnet, dass** die in Richtung senkrecht zur Längsausdehnung des Bandteils (1) gemessene vom Bandteil (1) seitlich abstehende Breite (b) des Hüllteils (18) mindestens 2cm und vorzugsweise höchstens 6cm beträgt.

10. Einrichtung nach einem der Ansprüche 1 bis 9, **dadurch gekennzeichnet, dass** das Hüllteil (18) aus einem elastischen Kunststoffmaterial, vorzugsweise aus Silikon besteht.

11. Einrichtung nach einem der Ansprüche 1 bis 10, **dadurch gekennzeichnet, dass** die Härte des Hüllteils (18) im Bereich zwischen 30 und 60 Shore A liegt.

12. Einrichtung nach einem der Ansprüche 1 bis 11, **dadurch gekennzeichnet, dass** das Hüllteil (18) mindestens eine Fensteröffnung (30) zum Annähen von über das angelegte Bandteil (1) gezogenem Magengewebe an unter der Fensteröffnung (30) liegendem Magengewebe aufweist.

13. Einrichtung nach einem der Ansprüche 1 bis 12, **dadurch gekennzeichnet, dass** das Hüllteil (18) zumindest auf der im angelegten Zustand des Magenbandes der Magenwand zugewandten Seite mit Titan beschichtet ist.

14. Einrichtung nach einem der Ansprüche 1 bis 13, **dadurch gekennzeichnet, dass** das Hüllteil (18) im angelegten Zustand des Magenbandes zumindest den zum Hüllteil (18) proximal liegenden Bereich des Magens (27) umgibt, vorzugsweise auch den ösophagealen-gastralen Übergang.

15. Einrichtung nach einem der Ansprüche 1 bis 14, **dadurch gekennzeichnet, dass** das Bandteil (1) mindestens eine mit einem Füllmedium befüllbare Hohlkammer (12) aufweist, die sich zumindest über einen Teil, vorzugsweise zumindest einen Großteil, der Länge des Bandteils (1) erstreckt, wobei sich bei einer Befüllung der Hohlkammer (12) ein die Magenbandöffnung (4) begrenzender innerer Abschnitt (14) des Bandteils (1) unter Verkleinerung der Magenbandöffnung in Richtung zur Achse der Magenbandöffnung (4) ausdehnt.

16. Einrichtung nach einem der Ansprüche 1 bis 15, **dadurch gekennzeichnet, dass** im geschlossenen Zustand der Verschlusseinrichtung (2, 3) das Hüllteil (18) ohne eine äußere Krafteinwirkung auf das Hüllteil (18) eine kuppel- bzw. domförmige Gestalt aufweist.

## Claims

1. A device for treating obesity, comprising a band part (1), placeable annularly around the stomach, with a closure device (2, 3) for closing the gastric band in the state placed annularly around the stomach and an enveloping part (18) for enveloping and supporting a stomach region lying proximally with respect to the applied band part (1), **characterised in that** the band part (1) and the enveloping part (18), secured to the band part (1) and projecting laterally therefrom, are in the form of a unit prefabricated by the manufacturer.

2. A device according to claim 1, **characterised in that** the enveloping part (18) has a plurality of through openings (19) distributed over its extension, wherein the sum of the areas of the through openings (19) preferably constitutes a majority of the total area of the enveloping part (18).

3. A device according to claim 2, **characterised in that** the enveloping part (18) is lattice-like.

4. A device according to any one of claims 1 to 3, **characterised in that** the enveloping part (18) is stuck to the band part (1), preferably in the region of one of the longitudinal edges (20, 21) of the band part (1).

5. A device according to any one of claims 1 to 4, **characterised in that** the two end-face ends (24, 25) of the enveloping part (18) are adjacent to one another in the state in which the gastric band is placed around the stomach or portions, adjoining the two end-face ends (24, 25), of the enveloping part (18) overlap one another, and the adjacent end-face ends (24, 25) or the overlapping portions adjoining the two end-face ends (24, 25) are securable to one another to form a circumferentially-closed supporting sleeve.

6. A device according to claim 5, **characterised in that** for mutual securing of the end-face ends (24, 25) or the overlapping portions, adjoining the end-face ends (24, 25), of the enveloping part (18), these can be sutured to one another, preferably by sewing eyelets (26).

7. A device according to claim 5 or claim 6, **characterised in that** a closure device is present for mutual securing of the end-face ends (24, 25) or the overlapping portions, adjoining the end-face ends (24, 25), of the enveloping part (18), which closure device preferably is formed by closure parts (31, 32) lockable to one another by engagement.

8. A device according to any one of claims 1 to 7, **characterised in that** the enveloping part (18) extends at least over the length of the band part (1).

9. A device according to any one of claims 1 to 8, **characterised in that** the width (b), measured in the direction perpendicular to the longitudinal extension of the band part (1) and projecting laterally from the band part (1), of the enveloping part (18) is at least 2 cm and preferably at most 6 cm.

10. A device according to any one of claims 1 to 9, **characterised in that** the enveloping part (18) is composed of a resilient synthetic material, preferably silicone.

11. A device according to any one of claims 1 to 10, **characterised in that** the hardness of the enveloping part (18) lies in the range between 30 and 60 Shore A.

12. A device according to any one of claims 1 to 11, **characterised in that** the enveloping part (18) has at least one window opening (30) for suturing stomach tissue drawn over the applied band part (1) to stomach tissue lying below the window opening (30).

13. A device according to any one of claims 1 to 12, **characterised in that** the enveloping part (18) is coated with titanium, at least on the side facing the stomach wall in the applied state of the gastric band.

14. A device according to any one of claims 1 to 13, **characterised in that** in the applied state of the gastric band, the enveloping part (18) surrounds at least the region, of the stomach (27), lying proximal to the enveloping part (18), preferably also the esophageal-gastric transition.

15. A device according to any one of claims 1 to 14, **characterised in that** the band part (1) has at least one hollow chamber (12) fillable with a filling medium, which hollow chamber (12) extends over at least a part, preferably at least a majority, of the length of the band part (1), wherein when filling the hollow chamber (12), an inner portion (14), bounding the gastric band opening (4), of the band part (1) expands in the direction of the axis of the gastric band opening (4), accompanied by a decrease in size of the gastric band opening.

16. A device according to any one of claims 1 to 15, **characterised in that** in the closed state of the closure device (2, 3), the enveloping part (18) has a cupola-shaped or dome-shaped form without external force effect upon the enveloping part (18).

## Revendications

1. Dispositif pour le traitement de l'obésité, comprenant une pièce de bande (1) de forme annulaire applicable autour de l'estomac avec un système de fermeture (2, 3) pour la fermeture de la bande gastrique en état d'application annulaire autour de l'estomac, et une pièce de gaine (18) destinée à envelopper et maintenir une partie de l'estomac disposée proximalement par rapport à la pièce de bande (1) appliquée, **caractérisé en ce que** la pièce de bande (1) et la pièce de gaine (18) fixée contre la pièce de bande (1) et s'étendant latéralement à partir de celle-ci sont réalisées comme une unité prête à l'emploi par le fabricant.

2. Dispositif selon la revendication 1, **caractérisé en ce que** la pièce de gaine (18) comporte une pluralité d'ouvertures de passage (19) réparties sur son extension, l'addition des surfaces des ouvertures de passage (19) représentant préférentiellement une grande partie de la surface totale de la pièce de gaine (18).

3. Dispositif selon la revendication 2, **caractérisé en ce que** la pièce de gaine (18) est réalisée en forme de réseau.

4. Dispositif selon l'une des revendications 1 à 3, **caractérisé en ce que** la pièce de gaine (18) est collée contre la pièce de bande (1), préférentiellement au niveau d'un des bords longitudinaux (20, 21) de la pièce de bande (1).

5. Dispositif selon l'une des revendications 1 à 4, **caractérisé en ce que** les deux extrémités frontales (24, 25) de la pièce de gaine (18) sont contiguës l'une à l'autre en état d'application de la bande gastrique autour de l'estomac, ou **en ce que** des parties adjacentes aux deux extrémités frontales (24, 25) de la pièce de gaine (18) se chevauchent l'une l'autre, et **en ce que**, pour former une manchette de maintien à périphérie fermée, les extrémités frontales (24, 25) contiguës l'une à l'autre ou les parties adjacentes aux deux extrémités frontales (24, 25) qui se chevauchent peuvent être fixées l'une contre l'autre.

6. Dispositif selon la revendication 5, **caractérisé en ce que**, pour une fixation mutuelle des extrémités frontales (24, 25) ou des parties adjacentes aux deux extrémités frontales (24, 25) de la pièce de gaine (18) qui se chevauchent, celles-ci peuvent être cousues l'une à l'autre, préférentiellement au moyen d'oeillets de couture (26).

7. Dispositif selon la revendication 5 ou la revendication 6, **caractérisé en ce que** pour une fixation mutuelle des extrémités frontales (24, 25) ou des parties adjacentes aux deux extrémités frontales (24, 25) de la pièce de gaine (18) qui se chevauchent, il est prévu un système de fermeture constitué préférentiellement de pièces de fermeture (31, 32) enclenchables l'une à l'autre.

8. Dispositif selon l'une des revendications 1 à 7, **caractérisé en ce que** la pièce de gaine (18) s'étend au moins sur la longueur de la pièce de bande (1).

9. Dispositif selon l'une des revendications 1 à 8, **caractérisé en ce que**, mesurée dans la direction perpendiculaire à l'extension longitudinale de la pièce de bande (1), la largeur (b) de la pièce de gaine (18) s'étendant latéralement à partir de la pièce de bande (1) est au minimum de 2 cm et préférentiellement de 6 cm au maximum.

10. Dispositif selon l'une des revendications 1 à 9, **caractérisé en ce que** la pièce de gaine (18) est composée d'une matière synthétique élastique, préférentiellement du silicone.

11. Dispositif selon l'une des revendications 1 à 10, **caractérisé en ce que** la dureté de la pièce de gaine (18) est comprise entre 30 et 60 Shore A.

12. Dispositif selon l'une des revendications 1 à 11, **caractérisé en ce que** la pièce de gaine (18) comporte au moins une ouverture de fenêtre (30) pour la couture du tissu gastrique tiré au-dessus de la pièce de bande (1) appliquée sur le tissu gastrique situé sous l'ouverture de fenêtre (30).

13. Dispositif selon l'une des revendications 1 à 12, **caractérisé en ce que** la pièce de gaine (18) est revêtue de titane au moins sur la face opposée à la paroi de l'estomac en état d'application de la bande gastrique.

14. Dispositif selon l'une des revendications 1 à 13, **caractérisé en ce que** la pièce de gaine (18) entoure au moins la partie de l'estomac (27) disposée proximalement par rapport à la pièce de gaine (18) en état d'application de la bande gastrique, de préférence également le passage oesophago-gastrique.

15. Dispositif selon l'une des revendications 1 à 14, **caractérisé en ce que** la pièce de bande (1) comporte au moins un compartiment (12) pouvant être rempli d'un milieu de remplissage et qui s'étend au moins sur une partie, préférentiellement au moins sur une grande partie de la longueur de la pièce de bande (1), une section intérieure (14) de la pièce de bande (1) limitant l'ouverture de la bande gastrique (4) se dilatant dans la direction de l'axe de l'ouverture de la bande gastrique (4) en cas de remplissage du compartiment (12), en réduisant l'ouverture de la bande gastrique.

16. Dispositif selon l'une des revendications 1 à 15, **caractérisé en ce qu'**en état fermé du système de fermeture (2, 3), la pièce de gaine (18) présente une forme en coupole ou en dôme, sans application extérieure d'effort sur la pièce de gaine (18).
